# EUROPEAN PATENT SPECIFICATION

(11) **EP 1 934 332 B1**
(45) Date of publication and mention of the grant of the patent: **23.02.2011**
(21) Application number: 06791427.5
(22) Date of filing: 28.09.2006
(51) Int. Cl.: C12N 5/07, A61K 6/00

(54) **METHODS FOR DIFFERENTIATING STEM CELLS AND USE THEREOF IN THE TREATMENT OF DENTAL CONDITIONS**
VERFAHREN ZUR DIFFERENZIERUNG VON STAMMZELLEN UND VERWENDUNG DAVON BEI DER BEHANDLUNG VON ZAHNLEIDEN
PROCEDE DE DIFFERENTIATION DES CELLULES SOUCHES ET SON UTILISATION POUR LE TRAITEMENT D'ETATS DENTAIRES

(30) Priority: 30.09.2005 US 722321 P
(43) Date of publication of application: 25.06.2008
(73) Proprietor: Global Dental ApS, DK 2300 Kobenhavn S. (DK)
(72) Inventor: OSTHER, Kurt, Scottsdale, AZ 85259 (US); CLAUSEN, Christian, DK-3480 Fredensborg (DK); PEDERSEN, Klaus Riskær c/o Iværksætterfabrikken, DK-2300 Copenhagen S (DK)
(74) Representative: Inspicos A/S
(86) International application number: PCT/DK2006/000533
(87) International publication number: WO 2007/036232

(56) References cited:
- KRAMER P R ET AL: "Mesenchymal stem cells acquire characteristics of cells in the periodontal ligament in vitro." JOURNAL OF DENTAL RESEARCH, vol. 83, no. 1, January 2004 (2004-01), pages 27-34, XP002410875 ISSN: 0022-0345
- GUAN GUOGIANG ET AL: "Adult stem cells acquire periodontal ligament characteristics in vitro" FASEB JOURNAL, vol. 18, no. 4-5, 2004, pages Abst. 66.7 URL-http://ww, XP002410876 & FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638
- TAKAT T ET AL: "ATTACHMENT, PROLIFERATION AND DIFFERENTIATION OF PERIODONTAL LIGAMENT CELLS ON VARIOUS GUIDED TISSUE REGENERATION MEMBRANES" JOURNAL OF PERIODONTAL RESEARCH, COPENHAGEN, DK, vol. 36, no. 5, October 2001 (2001-10), pages 322-327, XP001027431
- MIZUNO N ET AL: "Characterization of epithelial cells derived from periodontal ligament by gene expression patterns of bone-related and enamel proteins" CELL BIOLOGY INTERNATIONAL, ACADEMIC PRESS, GB, vol. 29, no. 2, February 2005 (2005-02), pages 111-117, XP004782085 ISSN: 1065-6995
- LEKIC P C ET AL: "Transplantation of labeled periodontal ligament cells promotes regeneration of alveolar bone" ANATOMICAL RECORD, A.R. LISS, NEW YORK, NY, US, vol. 262, no. 2, 1 February 2001 (2001-02-01), pages 193-202, XP002328906 ISSN: 0003-276X

## Description

### Field of the Invention

The present invention relates to a novel concept and methods for producing differentiated mammalian cells, which can be implanted for the repair of periodontitis, and other dental conditions, where these cells have characteristics and behaviour as differentiated periodontal ligament stem cells (e.g., PDLCs) or Periodontal ligament cells (PDLs). PDLs connect root cementum with alveolar bone, and are important for periodontal wound heating.

### Background of the invention

Bone marrow is suggested to be a predominant pool of a "stem cell preserve", containing not only haemopoietic stem cells (HSC), but also endothelial and mesenchymal stem cells (ESC, MSC). It is one of the target areas encompassed in this invention to obtain suitable cell material to be differentiated into both an appropriate autologous cell and/or an appropriate allogeneic cell that has been transformed to a cell type capable of being transplanted into a recipient without being rejected or causing a graft versus host reaction.

It is for instance conceivable that among other cell types that may be transformed by for instance by transdifferentiation, e.g., by co-culturing, as for instance the cells being co-cultured with periodontal ligament tissue or factors derived from the periodontal ligament tissue, any mesenchymal osteoblastic, or even precursors and progenitors of osteoblastic or fibroblastic lineages might be used to producer PDL cells.

Processes, compositions and uses of haematopoietic cells have previously been disclosed. Haematopoietic cells are cells, which can differentiate into mature blood cells when co-cultured with osteoblasts. Specifically, a process for propagating and maintaining the immature morphology of a haematopoietic cell by co-culturing the cells with osteoblasts as disclosed in US patent # 5733541.

The osteoblasts provide cytokines and/or a microenvironment which propagates and maintains the immature morphology of a haematopoietic cell. Haematopoietic cells are useful in the treatment of certain blood related disorders and are useful for treatment of patients in need of hematopoietic cells.

One of the aims of this present invention is actually not to maintain the immature morphology of hematopoietic cells, but rather to transdifferentiate mesenchymal stem cells (such as for instance mesenchymal stem cells from the umbilical cord or umbilical cord blood), osteoblasts and cells of lineages alike for instance by exposure to, or co-cultured in periodontal ligament tissue or factors from periodontal ligament tissue, in order to obtain a mesenchymal stem cell with characteristics such as showing increased expression of osteopontin and of osteocalcin, and at the same time, showing decreased expression of bone sialoprotein (BSP) resulting in a cell population resembling or alike PDL cells. It would be very uncommon in any other mesenchymal cell than periodontal ligament cells or periodontal ligament stem cells.

### Injection of multipotent adult progenitor cells

After injection of single multipotent adult progenitor cells into early blastocysts, the cells were found to contribute to most somatic cell types from all 3 germ layers. Moreover, bone marrow derived hematopoietic precursor cells appear to reside in non-hematogenic tissues, such as for instance muscle tissue as described by McKinney-Freeman et al. (McKinney-Freeman SL, Jackson, KA, et al., PNAS USA (2002) 99:1341).

Osteoblast-like cells have been observed within stromal layers and share several phenotypic characteristics with stromal cell lines (Benayahu D et al., Calcif Tissue Int. (1992), 51:195-201; Benayahu D et al., Calcif Tissue Int. (1991), 49:202-207; Mathieu E et al., Calcif Tissue Int. (1992), 50:362-371). For example, the murine bone marrow stromal cell lines BMS2 share a number of osteoblast markers including high alkaline phosphatase, collagen (I) and osteopontin as well as an increase in bone sialoprotein (BSP), which is not characteristic of a periodontal ligament stem cell or periodontal ligament cells. In addition, mRNA for osteocalcin, a osteoblast specific protein also was detected in BMS2 cells, a murine stromal cell line, also showing increased expression of bone sialoprotein, also called bone sialoprotein II pr (BSP) (Dorheim MA et al., J Cell Physiol. (1993)154:317-328); according to Dorheim et al., even pre-adipocytes and adipocytes may exhibit these osteoblastic characteristics.

In a series of experiments using several stromal cell lines, Benayhu et al found that all cell types examined (MBA-1: fibroblasts, MBA-2 endothelial-like, MBA-13 fibroendothelial, 13F1.1 cloned preadipocyte, MBA-15 osteoblastic) possess some osteoblastic features, but differed in the degree of expression (Benayahu D et al., Calcif Tissue Int. (1992), 51:195-201; Benayahu D et al., Calcif Tissue Int. (1991), 49:202-207). It has been found that recombinant human bone morphogenic protein-2 induces osteoblastic differentiation in the W-20-17 murine stromal cell line; and ectopic marrow transplantation experiments clearly demonstrate that newly formed bone marrow stroma and bone are derived from the donor, while blood cells are of host origin (Reddi AH, Coll Relat Res. (1981)1:209-226; Kataoka H, Urist MR, Clin Orthop Relat Res. (1993), 286:262-270).

Further evidence of a functional link between hematopoietic progenitor cells and osteoblasts is provided by observations that osteoprogenitor cells originate from the bone marrow. Several investigators have shown that both primary and transformed bone marrow stromal cells can acquire the osteoblast phenotype as bone formation is observed *in vivo* after implantation of these cells into diffusion chambers (Grigoriadis et al. J Cell Biol. (1988) 106:2139-51). In vitro incubation of non-adherent low-density bone marrow cells in serum free conditions can also develop into osteoblast-like cells, which can mineralize their extracellular matrix (Long et al., 1990 and Campbell et at., 1987).

The above literature describes essentially the osteoblast as a source of stimulating cells by producing cytokines and other factors that may aid in the particular stimulation of hematopoietic lineages.

The scope of this invention, however, is to create a cell type useable primarily for the repair of dental diseases (such as periodontitis) such as PDL cells or cells with PDL characteristics that either may be of allogeneic origin, mature or immature umbilical cord or umbilical cord blood.

### Summary of the invention

In one aspect, the present invention provides a process for propagating and/or differentiating mammalian mesenchymal cells from the umbilical cord or from the umbilical cord blood, the method comprising either 1) exposing mammalian mesenchymal cells from the umbilical cord or from the umbilical cord blood to periodontal ligament tissue or 2) co-culturing such mesenchymal cells with periodontal ligament tissue, to obtain cells having PDL characteristics and fulfilling at least one of the following: i) show periodontal characteristics as evidenced with Von Kossa method in which calcium phosphate deposits are stained brown to black, ii) show increased osteopontin and osteocalcin and at the same time decreased bone sialoprotein (bone sialoprotein II or BSP), iii) are capable of being implanted to repair and/or regenerate periodontal tissue, iv) are capable of repairing disorders such as paradentitis also called paradentosis, or periodontitis by healing of the gum line towards the teeth, and v) are accepted by the host without significant immune reaction or cell rejection.

This process results in mesenchymal cells having the capabilities regarding expression of proteins such as at least HLA-DR positive (HLA-DR+), CD-34 negative (CD-34 -), Lin positive (Lin +), Thy-1 positive (Thy-1+), Stro-1 positive (Stro-1 +), CD13 positive, CD44 positive, CD90 positive, CD73 positive and probably also CD146/MUC 18 positive (CD146/MUC 18 +). These stem cells or progenitors may be lesser immunogenic to a recipient due to the fact that they also are multipotential cells or mesenchymal stem cells or progenitors derived from umbilical cord or from umbilical cord blood.

### Disclosure of the invention

The present invention relates to a novel concept and methods for obtaining mammalian mesenchymal cell lines that have characteristics of PDL cells. The cells obtained are and will continue to be HLA-DR positive (HLA-DR+), CD-34 negative (CD-34 -), Lin-1 positive (Lin-1 +), Thy-1 positive (Thy-1+), STRO-1 positive (STRO-1 +), CD13 positive, CD44 positive, CD90 positive, CD73 positive and probably also CD146/MUC 18 positive (CD146/MUC 18 +). These cells are preferably mesenchymal cells from cord blood or preferably from human cord blood, obtained by co-culturing for instance co-cultured together with periodontal ligament tissue. Periodontal ligament cells and/or periodontal ligament stem cells could in theory be characterized by the expression of osteocalcin and osteopontin and most probably by a decreased expression or even lack of expression of bone sialoprotein, otherwise normally observed in osteogenic and in chondrogenic cell cultures.

The cells having PDL characteristics are suitable for use for the repair or treatment of dental disorders especially periodontits.

In order to obtain a predictable periodontal regeneration, selective adhesion and proliferation of PDL cells are essential. These types of cells may repair both impaired periodontal ligaments (e.g., periodontitis and disorders alike) and at the same time for instance produce cementum linking the fibrous tissue with the bone and may be capable of treating periodontal defects, thus re-establishing adhesion of teeth in danger of being lost due to periodontis.

According to this invention, these PDL cells, which first of are the specialized cells, which will be the starting material for the cell culture that can be used for the repair described above, such as for instance periodontitis, etc. It would of course be preferable, if these cells were autologous, meaning harvested from the teeth, such as the wisdom tooth at the same time as, it is extracted (so that the cells can be harvested immediately in conjunction with the extraction or the extracted tooth can be sent to the laboratory in Transport medium within for instance 24 hours and the PDL cells can be harvested and either cultured immediately or deepfrozen in liquid nitrogen tank for later use.

It may be possible that other cells could be used for the same purpose such as autologous or allogeneic osteoblasts, harvested from either the jaw bone or from other bone structure on the patient. These mesenchymal precursor cells or progenitor cells, might also be used for the purpose of repairing periodontitis, etc. Yet another group of cells, which may be derived from a multipotent mesenchymal stem cell originated from the umbilical cord or the umbilical cord blood, which either could be derived as an autologous cell (taken from stored umbilical cord blood and used to - later in life - repair periodontitis in a patient that donated his/her umbilical cord blood at the time of birth. Or these multipotent mesenchymal stem cells from umbilical cord from other individuals may show lesser tendency to be immunogenic, and therefore useable as "donor" cells to repair periodontitis etc. When such cells are used they have been subjected to a co-culturing process, wherein the cells have been exposed to or cultured together with periodontal ligament tissue, in order to obtain PDL characteristics.

One could co-culture cells suitable to act as PDL cells or PDLCs with other mammalian mesenchymal precursor cells (e.g., any mamalian mesenchymal stem cells such as adult mesenchymal precursor cells, The PDL cells might also for instance be co-cultured with multipotent mesenchymal stem cells for instance such stem cells originated from umbilical cord or from umbilical cord blood, and in this manner, one can obtain a co-cultured cell suitable for use in the repair of periodontitis, etc.

In order to obtain a periodontal ligament stem cell population, one may co-culture the mesenchymal stem cells with periodontal ligament tissue to differentiate or transdifferentiate into PDL cells. It is anticipated that the culturing may range from a short duration exposure of the cells to the periodontal ligament tissue or proteins, to a complete expansion of a cell culture in culture medium that at least contains periodontal ligament tissue, lasting several weeks until a sufficient amount of cells have been obtained. This cell culturing methodology is used on mesenchymal stem cells derived from the umbilical cord and/or from the umbilical cord blood. Thus mesenchymal stem cells from the umbilical cord or from the umbilical cord blood, together with periodontal ligament tissue may induce mesenchymal stem cells to obtain periodontal-ligament-like characteristics and thus be useable as cell implants with or without a scaffold in the clinical application for repairing and regenerating periodontal tissue.

### Starting material - mammalian mesenchymal cells

The mesenchymal stem cells may be differentiated to PDLs or PDLCs by exposing these cells to and/or co-culture them with periodontal ligament tissue which may induce the precursor cells, or keep cell such as PDLs from de-differentiating.

It should therefore be understood and be within the scope of this invention that the cell types described above, may, when exposed to, co-cultured with and/or induced by periodontal ligament tissue or factors derived from periodontal ligament be differentiated or transdifferentiated to PDLs or PDLCs. The same harvested periodontal ligament tissue may, when used in the medium, when using the medium for co-culturing mesenchymal stem cells from the umbilical cord, result in PDLs or cells having the same abilities as PDLs, to enable the use of them for the treatment of periodontitis, etc.

Using the methods described above, may also enable the inventors to culture osteoblasts or progenitors of osteogenic cells, as for instance obtained by explantation of biopsies from a mammalian, for instance from the bone, especially bone structures obtained from the patient to be developed to PDLs or cells with PDL behaviour for the treatment of periodontitis later on in the patient's life, meaning in this manner utilizing an autologous cell implantation methodology, when one has no access to primary cultures of PDLs.

### Periodontal ligament tissues used in the cell culture medium as inducer of PDL.

### Source of periodontal ligament tissue

The source of periodontal ligament tissue may be of autologous, allogeneic or xenogeneic origin (e.g., extracted or avulsed porcine teeth). However, it is anticipated that the processing of either cells from periodontal ligament, the periodontal ligament or factors have to be harvested and processed, while the tissue is still viable and has not undergone disintegration of any of the necessary ingredients needed for obtaining and possibly for maintaining the cultured cells as PDLs or PDLCs or cells expressing themselves as PDLs or PDLCs. Suggestions for handling the periodontal tissue is described under "Harvest and preservation of periodontal tissue".

The periodontal ligament to be used in the culture medium to induce "PDL effect" in the cultured cells in question, can be harvested for instance from molar teeth or from wisdom teeth. So, the periodontal ligament may either be harvested together with the PDL cells already incorporated in the periodontal ligament, or the periodontal ligament tissue may be depleted from these cells, and consist of periodontal ligament tissue for future use in PDL cell culturing or in the attempts to transdifferentiate the other mesenchymal cells described above to be PDLs.

This "periodontal ligament tissue", which according to this invention may be defined as an inducing mixture to add to a mesenchymal cell culture (intended to be used as PDL cells). This may be stored deep frozen (e.g., for instance in a nitrogen tank), or used it may be used directly mixed together with a suitable growth medium (culture medium) in which the cells are to be cultured for the purpose of differentiating or transdifferenting other selected cells to become or to resembleo PDLs or PDLCs. Said medium composition containing the periodontal ligament tissue or factors from periodontal ligaments can accordingly be used as growth medium, which will maintain PDL cells and thus avoid de-differentiation or cell "drifting".

As described previously this periodontal ligament tissue, (for instance depleted from cells), may also be collected, characterized and pooled, either to be used together with culture medium to culture mesenchymal cells in order to induce PDL cell characteristics resulting in said cell population that may be characterized as a periodontal ligament cell or a precursor cell of said type. The PDL cell characteristics obtained by differentiation from mesenchymal cells by the inductive use of the periodontal ligament tissue can be demonstrated by showing that said PDL or PDL like cells will show increased expression of osteopontin and osteocalcin and decreased expression of bone sialoprotein (decreased BSP expression), also called bone sialoprotein II.

### Harvest and preservation of periodontal tissue

In order to utilize periodontal tissue in the amounts that may be needed for the induction, transdifferentiation or transformation of mesenchymal cells to produce periodontal ligament cells or cells that may behave like periodontal ligament cells, it may be necessary to prepare a logistic system to keep cells of the teeth extracted alive (e.g., extracted wisdom teeth collected from various dental clinics) as well as keeping the periodontal tissue from the extracted teeth from disintegrating, most probably meaning that the extracted teeth be kept alive and in aseptic conditions to prevent microbial contamination (microbial contamination could be kept at a minimum by using as aseptic methods as possible and add sufficient amounts of antibiotics and antifungals present in the transport or culture medium used for storage). Sigalas et al. have recently tested methods to keep the cells of avulsed teeth alive. Many solutions have been examined as possible storage media for avulsed teeth. Sigalas et al has recently reported that when human periodontal ligament (PDL) cells were exposed for 1 h to culture medium, milk, Hanks Balanced Salt Solution (HBSS), Soft Wear, Opti Free, and Solo Care contact lens solutions, Gatorade, and tap water, at room temperature and on ice. The number of viable cells was counted using the trypan blue exclusion technique, immediately after exposure (0 h) and at 24 and 48 h, the proliferative capacity of the cells was tested after treatment (Sigalas E, Regan JD et al. Dent. Traumatol. (2004) 20:21-28). This study showed that Hanks Balanced Salt Solution HBSS was found to be the optimal storage medium for avulsed teeth. It is conceivable that many other types of culture medium with or without the addition of periodontal ligament tissue present from the start, and most probably containing serum proteins. The teeth, the periodontal ligament related cells or the periodontal ligament tissue may be deep-freezed in a nitrogen tank.

The manner in establishing that cells have not differentiated into PDL or that PDL cells have de-difffentiated or drifted the the bone sialoprotein (also named bone sialoprotein II or BSP) will be increased and actually demonstrate that the cells cultured in fact are osteoblasts and certainly not PDL cells. Actually, when the sialoprotein II or BSP can be identified (or shown to increase) one is aware that the PDL cell culture has changed into osteogenic cells (e.g., osteoblasts and non PDL- or non PDLC-differentiated mesenchymal cells (Fisher LW, McBride OW, et al. J. Biol. Chemistry (1990), 265:2347-2351). The BSP protein encoded by this gene is a major structural protein of the bone matrix. It constitutes approximately 12% of the noncollagenous proteins in human bone and is synthesized by skeletal-associated cell types, including hypertrophic chondrocytes, osteoblasts, osteocytes, and osteoclasts. This protein binds to calcium and hydroxyapatite via its acidic amino acid clusters, and mediates cell attachment through an RGD sequence that recognizes the vitronectin receptor. The BSP gene is also identified as geneID: 3381 and expresses integrin-binding sialoprotein (bone sialoprotein, also called bone sialoprotein II, abbreviated to IBSP).

We have previously found by Affymetrix Gene Chip analysis that the expression of vitronectin receptor also is presented in chondrocytes from both Osteoarthritis patients and non-O.A. patients (own observations). However, this expression has as of yet not been tested in PDLs or PDLCs.

Cells implanted for the periodontal repair and/or dental repair may be autologous mesenchymal stem cells, precursor cells or progenitors as for instance harvested as stem cells or precursors from periodontal areas or periodontal ligament(s), or even mesenchymal cells originated from jaw bone biopsies can be differentiated towards PDLs when exposed to periodontal ligament tissue. Therefore we may in theory and actually in practice use the explant culture technology to espand mesenchymal cells or rather osteoblasts or precursors thereof to produce PDL cells. These PDL cells can then be used for implantation in the patient to repair periodontitis, etc.

These cells may be expected to further differentiate into mammalian mesenchymal cells, which may be encompassed in the rather complex process, producing a cell identity that resembles mammalian mesenchymal cell lines, which can be identified by continuing to be at least HLA-DR positive (HLA-DR+), CD-34 negative (CD-34 -), Lin-1 positive (Lin-1 +), Thy-1 positive (Thy-1+) and Stro-1 positive (Stro-1 +), CD13 positive, CD44 positive, CD90 positive, CD73 positive and possibly, positive for another protein, CD146/MUC18, but negative for CD31 (a haematopoietic marker).

It is within the scope of this invention to arrive at one or several mammalian cell types that can be transplanted into mammalians including humans without causing rejection of the transplanted or implanted cells or produce a "graft vs. host" (GVH) rejection, neither becoming apoptotic or inducing apoptosis or, when transplanted, without turning into immortalized cell lines.

When using embryonal mesenchymal stem cells for implantation for instance in joints, in attempts to develop methods for cartilage or O.A. repair, such implantation with embryonal mesenchymal stem cells have shown that these cells may retain the function of transforming during differentiation into mixed endoderm- mesoderm-ectoderm cells with immortalized behaviour appearing as teratomas in a significant amount of SCID mice under experiments, where said embryonal stem cells actually induced these immortalized teratomas (Wakitani S, Takaoka K, Hattori T, Miyazawa N, Iwanaga T, Takeda S, Watanabe TK and Tanigami A, Rheumatology (2003), 42:162-165) indicating that when using more immature cells, for instance for the purpose of producing periodontal ligament cells, it more advisable to use mesenchymal stem cells from the umbilical cord or from umbilical cord blood instead of using embryonal, stem cells for this purpose, because such transdifferentiation may actually produce tumors, for instance such as teratomas.

One of the main limiting factors for increased use of human umbilical cord blood (UCB) in adult allogeneic transplantation is the small number of progenitor cells that can be collected and infused. *Ex* v*ivo* expansion of UCB might help to overcome this limitation. Whether culturing of UCB cells may also lead to co-expansion of contaminating maternal cells, and thus altering the graft characteristics leading to an increased incidence of cell rejection, has not been looked at so far, but should be taken into consideration when using mesenchymal stem cells from the umbilical cord blood.

This could for instance be investigated by probing the method of isolating mesenchymal stem cells such as UCB cells, by initiating cultures of UCB mononuclear cells (MNC) in a standard medium containing stem cell factor (SCF), flt-3L, II-3, IL-6, EPO and G-CSF. To address the question of contaminating maternal cells one may perform a so-called interphase FISH analysis of the X and Y chromosome simultaneously. Male (XY) cord blood samples can be investigated for maternal (XX) cells at day 0 and at several time points during culture.

### Method for obtaining PDL cells

Mammalian mesenchymal cells are cultured together with periodontal ligament tissue. These cells are checked and analyzed on flow cytometry using conjugated monoclonal antibodies against; CD13, CD44, CD90, CD73 and CD31. Markers such as CD13, CD44, CD90, CD73 are all considered to be mesenchymal stem cell markers while CD31 is a haematopoietic marker. hPDLCs were demonstrated to be positive for all four stem cell markers, while negative for CD31.

The periodontal ligament tissue employed is first subjected to culturing using a suitable medium e.g. as described in Examples 1 or 6 herein. The growth medium is changed regularly, e.g. every 1-5 days such as every 3-4 days during the whole culture period. After a period of time (normally 8-11 days) cells (hPDLCs) migrate from the ligament and when sufficient confluent, the cells are harvested, e.g. using enzymatic treatment like trypsin/EDTA treatment.

Mammalian cells and hPDLCs cells were cultured in suitable culture dishes and fed with growth medium (e.g. DMEM/F12, containing 16% FCS, ascorbic acid, gentamicin and fungizone) at 37°C and 5% CO₂. Growth medium was changed every 3-4 days during the whole culture period.

After 8-11 days cells (called hPDLCs) start in general to migrate out from the small pieces of periodontal ligaments. When the culture was 70-80% confluent (approx. 3 weeks) cells were detached using trypsin/EDTA treatment and used for the experiments described below.

Alternatively, mammalian cells can be cultured in suitable culture dishes in the above described DMEM/F12, containing FCS, and other ingredients described above, with medium change every 3 to 4 days during the entire cell culture period, after a period of 11 days or more, such factors as for instance periodontal ligament proteins can be added as inducers in cells, where one attempt to transdifferentiate these to PDLs.

### Scaffolds

Bone cells or osteogenic cells cultured on various biodegradable scaffolds may create interaction between scaffold and the cells, when allowing them to adhere during transplantation or implantation. This may be accomplished among others, by using various bioresorbable polymers such as PLGA scaffolds.

It will be anticipated that several types of scaffolds may be tested and may capable of being used successfully for periodontal ligament cells and/or periodontal ligament stem ' cells or mesenchymal stem cells, which has been induced to be used as periodontal ligament tissue.

The cells to be implanted may be implanted together with a scaffold such as for instance a hydroxyl apatite carrier, which may enhance both a periodontal ligament and even cementum as well as producing a fibrous structure such as resembling Sharpey's fibers, which insert into both the cementum and bone while holding the teeth in place.

### Use of transplanted or implanted cells for the treatment of periodontitis

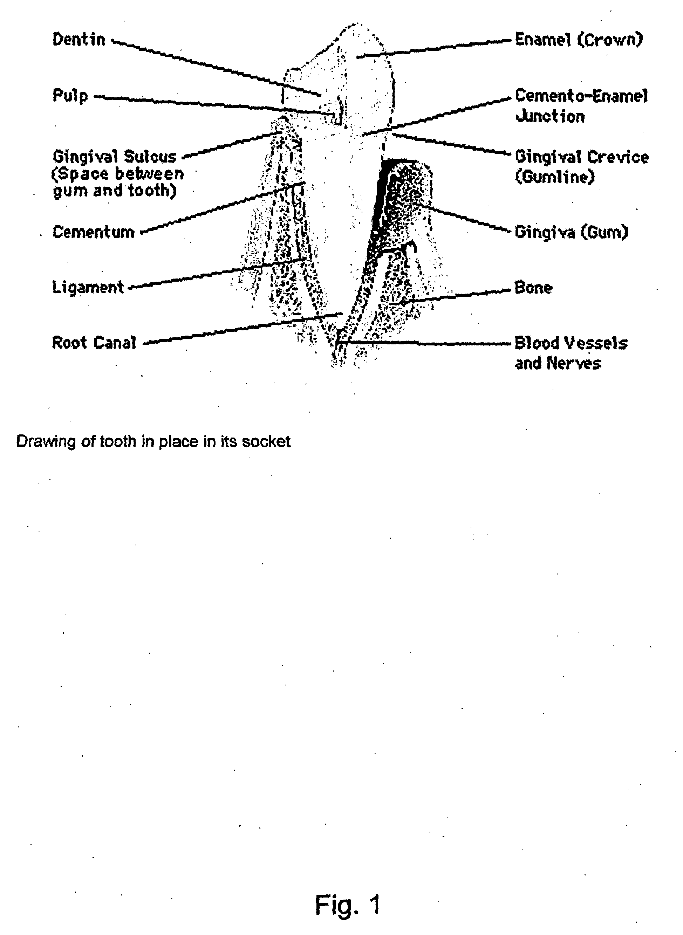

One of the purposes of using differentiated or transdifferentiated cells are to develop a cell or tissue material that can be used to for instance to treat destroyed periodontal tissue, which among others are caused "periodontitis". The oral disorder, called periodontitis, normally starts up as an infection induced by a microbial (e.g., bacterial or fungal) infection for instance in the gumline, diagnosed as a gingivitis or an acute gingivitis. This type of infection may initially seem harmless, but during time this infection may lead to necrosis of tissue essential for the oral cavity, and especially of the periodontal ligament, binding the teeth to the alveolar bone (together with cementum). This necrosis is caused among others by increased synthesis of tissue-destructive enzymes such as for instance collagenase, as well as activation of bone tissue decay caused by osteoclasts. These processes are capable of destroying those tissue structures, which normally keep the teeth anchored in the bone in the upper - and lower jaw (see Figure 1).

These methods could change the treatment of periodontitis considerably to a cell implantation repair, possibly together with a scaffold.

The root of the tooth fits precisely into the "pocket" in the bone structure (the alveolar bone) in healthy periodontal tissue (see Figure 1). The tooth is held *in situ* by the periodontal ligaments.

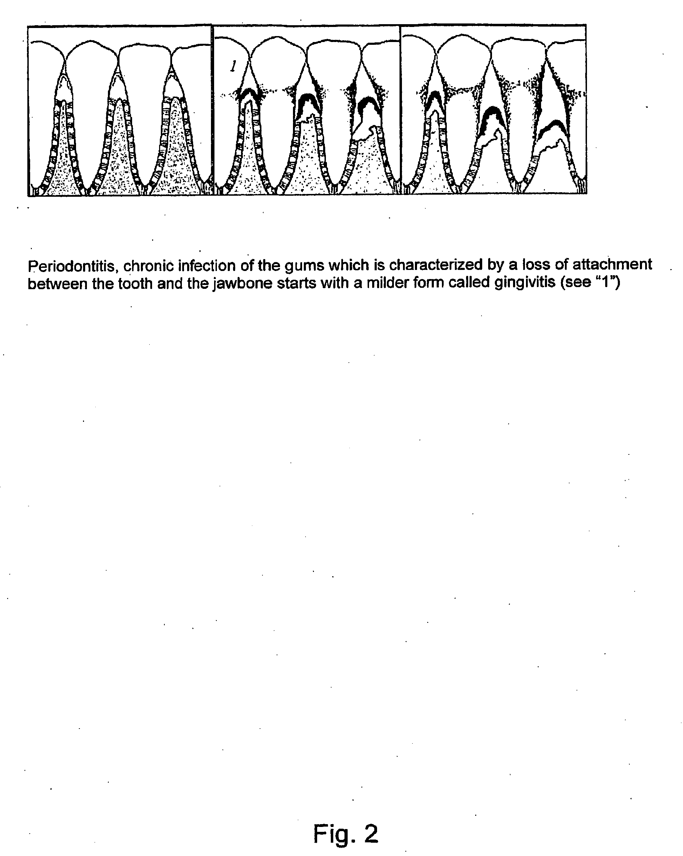

During periodontitis it is actually the periodontal ligaments, which are affected and eventually will result in tooth loosening as shown in Figure 2.

The cell treatment is accordingly based upon *ex vivo* cell culturing as described above and provides lesser tendency to reactions such as for instance rejection of the cells, or be of autologous origin and therefore show no evidence of rejection.

It is also within the scope of this invention to combine the cells to be implanted with appropriate scaffolds, so that one can shape and fit the product intended for the particular area of application, and even to cover the transplanted cells, to maintain their function, protect them against infection and even to limit the cells from growing into unwanted areas in the oral cavity.

### Legends to figure

Figure 1. Drawing of tooth in place in its socket
Figure 2. Periodontitis, chronic infection of the gums which is characterized by a loss of attachment between the tooth and the jawbone starts with a milder form called gingivitis (see "1")
Figure 3. A subconfluent culture of hPDLCs are shown. When the cells are 70% confluent, they are trypsinized
Figure 4. The samples were analyzed on a Beckman Coulter Epics XL flowcytometer. Dead cells and debris were excluded from the analysis by forward -and sidescatter gating
Figure 5. The cells were stained for alkaline phosphatase activity, studied using a specific staining protocol and the osteogenic differentiation is shown in this figure

The invention is illustrated in the following non-limiting examples

### Example 1

### hPDLC Culture

Human third molars were placed in Petri dishes, and 2 ml growth medium (DMEM/F12 containing 16% FCS, Gentamicin, Ascorbic Acid and Fungizone) were applied to the molars.

The periodontal ligament was gently separated from the surface of the root with a sterile scalpel and the tissue was transferred to a new petri dish.

2 ml growth medium were applied to the periodontal ligament tissue and small explants were generated with a sterile scalpel.

The explants from the periodontal ligament tissue were transferred to a 75 cm² culture flask containing growth medium and cultured at 37°C in a CO₂ incubator. The explants produced cell cultures in colony forming units from the primary cell culture were isolated.

### Example 2

### Surface Marker Expression

hPDLCs from the primary culture (E₁P₀) (defined as the initial CFU of cells primarily being grown out from the explant, which have not been passaged yet) were analyzed on flow cytometry using conjugated monoclonal antibodies against; CD13, CD44, CD90, CD73 and CD31.

CD13, CD44, CD90, CD73 are all considered to be mesenchymal stem cell markers while CD31 is a haematopoietic marker.

hPDLCs were demonstrated to be positive for all four stem cell markers, while negative for CD31.

This suggests that the hPDLC population is composed of mesenchymal progenitor cells.

### Example 3

### Osteogenic Differentiation (Gene expression)

hPDLCs were seeded in 6-well plates and induced to differentiate along the osteogenic pathway by changing the medium from normal growth medium to osteogenic medium (DMEM/F12 containing 10% FCS, Ascorbic Acid, 10⁻⁷M dexamethasone, 8 mM □-Glycerophosphate).

As control, hPDLCs were cultured in normal growth medium containing 10% FCS.

After 14 days in culture, hPDLCs were washed in PBS and RNA was isolated from the induced cultures and from the control cultures.

By using RT-PCR the expression of alkaline phosphatase (ALP), collagen type 1 (Col1) and osteocalcin (OC) were evaluated.

### Example 4

### Osteogenic Differentiation (Histochemical analysis)

hPDLCs were seeded in 6-well plates and induced to differentiate along the osteogenic pathway by changing the medium from normal growth medium to osteogenic medium (DMEM/F12 containing 10% FCS, Ascorbic Acid, 10⁻⁷ M dexamethasone, 8 mM □-Glycerophosphate). As control, hPDLCs were cultured in normal growth medium containing 10% FCS.

After 14 days in culture, hPDLCs were washed once in PBS. Subsequently, induced and control cultures were stained for either ALP activity or the presence of calcium deposits (Von Kossa staining).

Both an increased ALP activity and an increased calcium deposition were demonstrated for the induced cultures supporting the gene expression results.

All three markers were up-regulated in the induced cultures; demonstrating that hPDLCs were able to differentiate along the osteogenic pathway, suggesting that this cell population consists of immature mesenchymal stem cells.

### Example 5

### Freezing of hPDLCs

hPDLCs were trypsinized and filtrated through a 70 µm sterile filter and then centrifuged.The pellet was resuspended in freezing medium (FCS + 10% DMSO) and the cells were transferred to a cryo-vial. A graduated freezing process were initiated, starting at 4 °C for 1 hour, -20°C for 4 hours, -80 °C overnight and finally to liquid N₂.

### Example 6

Normal human third molars were collected immediately following extraction from 3 individuals. Periodontal ligaments were gently separated from the surface of the root, and they were then minced into small pieces. They were then transferred to T-75 culture flasks and fed with growth medium (DMEM/F12, containing 16% FCS, ascorbic acid, gentamicin and fungizone) at 37°C and 5% CO₂. Growth medium was changed every 3-4 days during the whole culture period.

After 8-11 days cells (called hPDLCs) started to migrate out from the small pieces of periodontal ligaments. When the culture was 70-80% confluent (approx. 3 weeks) cells were detached using trypsin/EDTA treatment and used for the experiments described below.

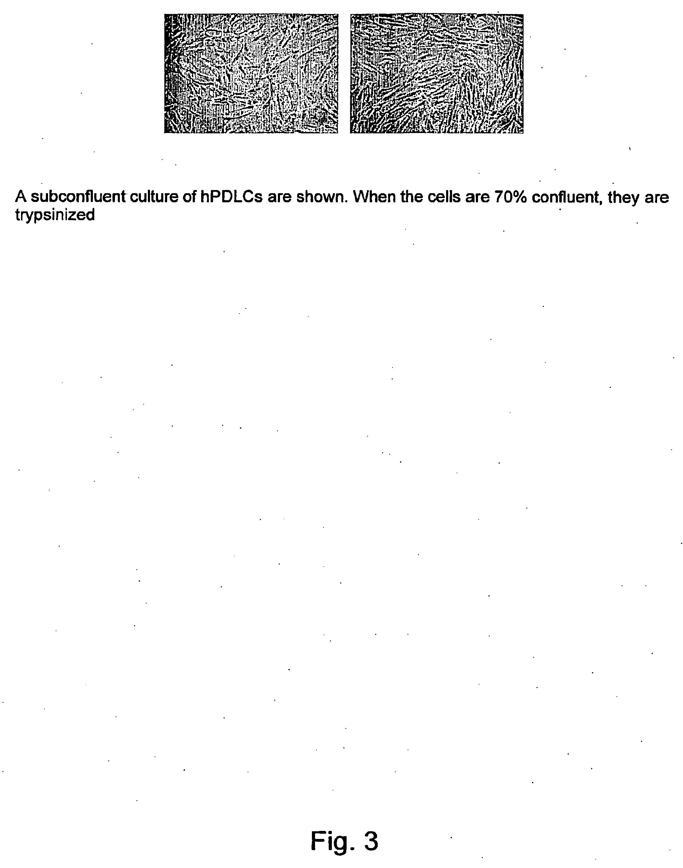

A subconfluent culture of hPDLCs are shown on Figure 3.

### Flow cytometry analysis

Flow cytometry analysis was performed in order to study the stem cell properties of hPDLCs.

Single cell suspensions of hPDLCs were incubated at room temperature with FITC-conjugated monoclonal antibodies against CD73, CD90, known as mesenchymal stem cell markers. After incubation, samples were analysed on a Beckman Coulter Epics XL flowcytometer. Dead cells and debris were excluded from the analysis by forward -and sidescatter gating.

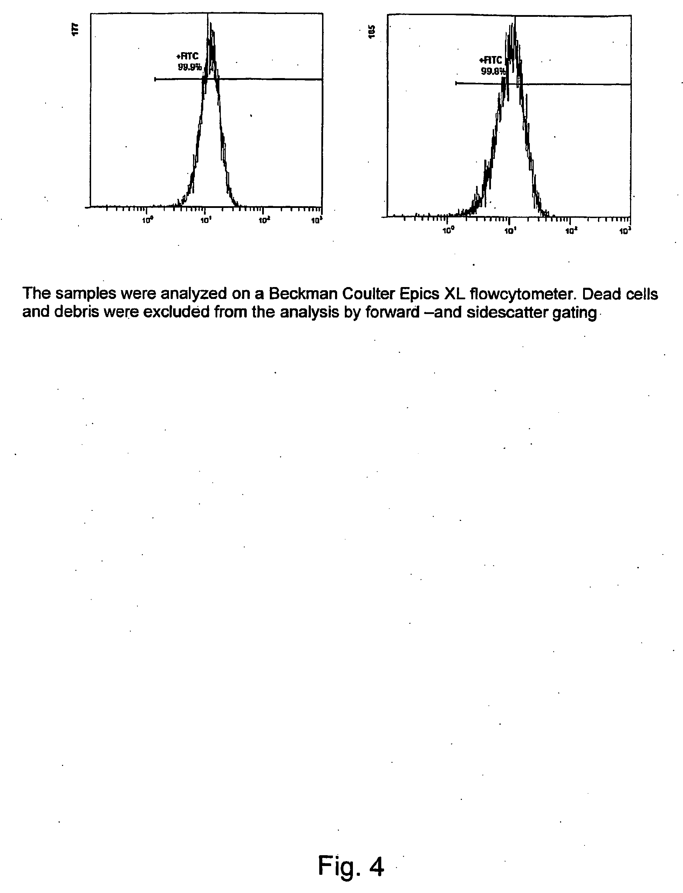

The results are shown on Figure 4.

### Osteogenic differentiation

Cells were seeded at 0.5x10⁴ cells/cm² and cultured overnight in growth medium. Osteogenic differentiation was induced for 3 weeks by shifting the cells from growth medium to an osteoinductive medium (OIM), composed of DMEMF12 with 10% FBS, 85 □g/mL L-ascorbic acid 2-phosphate, 10⁻⁷ M dexamethasone, and 8 mM β-glycerophosphate. Control cultures without the differentiation stimuli were cultured in SCM containing 10% FBS. Media changes were done twice weekly.

Calcification of the extracellular matrix (ECM) can be detected by the Von Kossa method in which calcium phosphate deposits are stained brown to black. Cells were seeded in 6-well plates at a density of 0.5x10⁴ cells/cm² and cultured for 21 days in OIM. In addition the alkaline phosphatase activity was studied using a specific staining protocol.

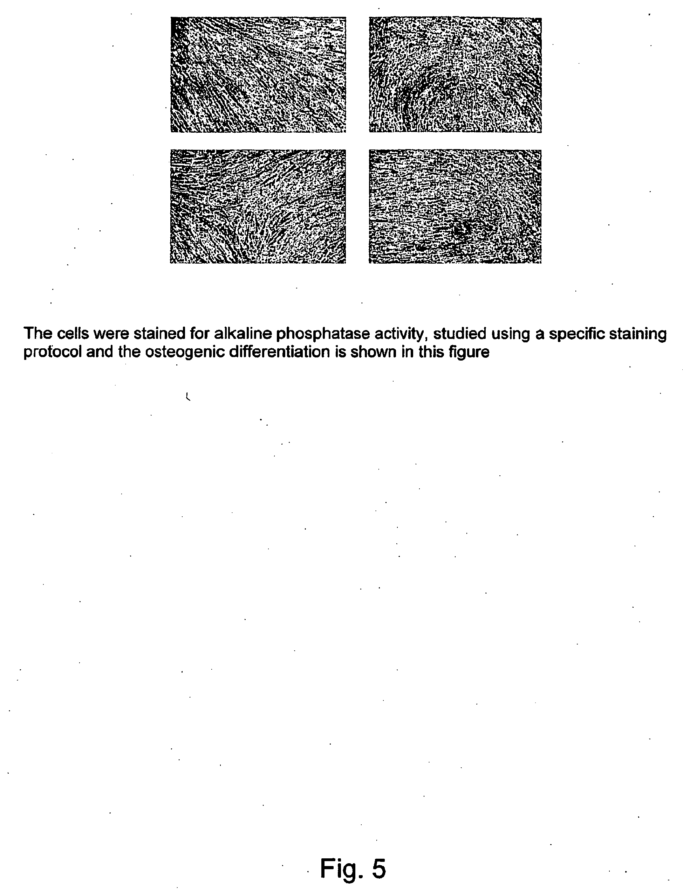

Results from the osteogenic differentiation are shown on Figure 5.

Based on the findings above we conclude that hPDLCs cultured in the above described growth medium arse able to differentiate along the osteogenic pathway.

Furthermore this cell population does display two surface markers, characteristic for mesenchymal stem cell.

Specific embodiments also described herein
1. A process for propagating mammalian mesenchymal cells, that have been exposed to and/or co-cultured with periodontal ligament tissue, periodontal ligament proteins or factors derived from periodontal ligament tissue and induced by these factors to
   a. Obtain periodontal characteristics
   b. Show increased osteopontin and osteocalcin and a decrease in bone sialoprotein, also called bone sialoprotein II or BSP
   c. Be capable of being implanted to repair and/or regenerate periodontal tissue
   d. Capable of repairing disorders such as paradentitis also called paradentosis
   e. Be accepted by the host without significant immune reaction or cell rejection
2. A process for progating allogenic mammalian mesenchymal cells derived from umbilical cord and/or umbilical cord blood, and which has been exposed to or co-cultured with periodontal ligament tissue, periodontal ligament proteins or factors derived from periodontal ligament tissue and induced by these factors to
   a. Obtain periodontal characteristics
   b. Show increased osteopontin and osteocalcin and a decrease in bone sialoprotein
   c. Be capable of being implanted to repair and/or regenerate periodontal tissue
   d. Capable of repairing disorders such as paradentitis also called paradentosis
   e. Be accepted by the host without significant immune reaction or cell rejection
3. A process for propagating autologous mesenchymal cells that have been exposed to or co-cultured with periodontal ligament tissue, periodontal ligament proteins or factors derived from periodontal ligament tissue and induced by these factors to
   a. Obtain periodontal characteristics
   b. Show increased osteopontin and osteocalcin and a decrease in bone sialoprotein
   c. Be capable of being implanted to repair and/or regenerate periodontal tissue
   d. Capable of repairing disorders such as paradentitis also called paradentosis
   e. Be accepted by the host without significant immune reaction or cell rejection
4. A process for propagating autologous osteoblasts or precursor/progenitor cells that have been exposed or co-cultured with periodontal ligament tissue, periodontal ligament proteins or factors derived from periodontal ligament tissue and induced by these factors to
   a. Obtain periodontal characteristics
   b. Show increased osteopontin and osteocalcin and a decrease in bone sialoprotein
   c. Be capable of being implanted to repair and/or regenerate periodontal tissue
   d. Capable of repairing disorders such as paradentitis also called paradentosis
   e. Be accepted by the host without significant immune reaction or cell rejection
5. A process for propagating cells as described in items 1 - 4 and being injected directly down to the remaining periodontal ligaments
6. A process of propagating autologous mesenchymal cells to be directly injected into the periodontal ligament *in situ,* thereby be capable of transdifferentiate *in situ*
7. A process for propagating and maintaining the immature morphology of mammalian cells, said immature morphology being defined as HLA-DR positive (HLA-DR+), Lin positive (Lin +), Thy-1 positive (Thy-1+), Stro-1 positive (Stro-1 +), CD-13 positive, CD-44 positive, CD-90 positive, CD-73 positive and, possibly CD146/MUC 18 positive shall be
   a. capable of being co-cultured with mammalian mesenchymal stem cells from umbilical cord
   b. capable of being co-cultured with mammalian mesenchymal stem cells from umbilical cord blood
   c. Be capable of being implanted to repair and/or regenerate periodontal tissue
   d. Capable of repairing disorders such as paradentitis also called paradentosis
   e. Be accepted by the host without significant immune reaction or cell rejection
8. A process for propagating and maintaining the immature morphology of mammalian cells, said immature morphology being defined as HLA-DR positive. (HLA-DR+), CD-34 negative (CD-34 -), Lin positive (Lin +), Thy-1 positive (Thy-1+), Stro-1 positive (Stro-1 +), CD-13 positive, CD-44 positive, CD-90 positive, CD-73 positive and, possibly CD146/MUC 18 positive shall be
   a. capable of being co-cultured with mammalian mesenchymal stem cells from umbilical cord
   b. capable of being co-cultured with mammalian mesenchymal stem cells from umbilical cord blood
   c. Be capable of being implanted to repair and/or regenerate periodontal tissue
   d. Capable of repairing disorders such as paradentitis also called paradentosis
   e. Be accepted by the host without significant immune reaction or cell rejection
9. capable of being co-cultured with mammalian cells derived from bone marrow cells or colonies thereof from either autologous or from allogeneic donors, exposed or co-cultured with periodontal ligament tissue, periodontal ligament proteins or factors derived from periodontal ligament tissue and induced by these factors to
   a. Obtain periodontal characteristics
   b. Show increased osteopontin and osteocalcin and a decrease in bone sialoprotein
   c. Be capable of being implanted to repair and/or regenerate periodontal tissue
   d. Be capable of being implanted to repair and/or regenerate periodontal tissue
   e. Capable of repairing disorders such as paradentitis also called paradentosis
   f. Be accepted by the host without significant immune reaction or cell rejection
10. A process for propagating and maintaining the immature morphology of mammalian cells, said immature morphology being defined as HLA-DR positive (HLA-DR+), Lin positive (Lin +), Thy-1 positive (Thy-1+), Stro-1 positive (Stro-1 +), CD-13 positive, CD-44 positive, CD-90 positive, CD-73 positive and, possibly CD146/MUC 18 positive the process comprising part of the identity of mammalian periodontal ligament cells can be
   a. capable of being co-cultured with mammalian mesenchymal stem cells from umbilical cord
   b. capable of being co-cuftured with mammalian mesenchymal stem cells from umbilical cord blood
   c. Be capable of being implanted to repair and/or regenerate periodontal tissue
   d. Capable of repairing disorders such as paradentitis also called paradentosis
   e. Be accepted by the host without significant immune reaction or cell rejection
11. The resulting population processed as described in 1, and 2, defined as HLA-DR positive (HLA-DR+), Lin positive (Lin +), Thy-1 positive (Thy-1+) and Stro-1 positive (Stro-1 +), CD-13 positive, CD-44 positive, CD-90 positive, CD-73 positive and, possibly CD146/MUC 18 positive shall be transplantable into mammals
   a. into their oral cavity without being rejected
   b. into the periodontal area without being rejected
   c. into dental defects without being rejected
12. The resulting population processed as described in 1, and 2, defined as HLA-DR positive (HLA-DR+), Lin positive (Lin +), Thy-1 positive (Thy-1+) and Stro-1 positive (Stro-1 +), CD-13 positive, CD-44 positive, CD-90 positive, CD-73 positive and, possibly CD146/MUC 18 positive shall be transplantable into mammals
   d. into their oral cavity without being rejected
   e. into the periodontal area without being rejected
   f. into dental defects without being rejected
13. The method of implanting cells as described in items 1 - 11 using a combination of cells and scaffold(s)
14. A process for propagating mammal Autologous Periodontal Ligament Positive Cells (stem cells) to be transplantable
   a. Into an oral cavity without being rejected
   b. Into the periodontal area without being rejected
   c. Into dental defects without being rejected.
15. The method of implanting cells as described in the claims above using a combination of cells and scaffold(s).
16. The use of periodontal ligament or extracts thereof from either autologous, allogeneic and/or xenogeneic source to enable cells to differentiate.
17. The use of periodontal ligament or extracts thereof from either autologous, allogeneic and/or xenogeneic source to enable cells to differentiate into periodontal cells, periodontal stem cells and/or cells expressing periodontal behavior.

## Claims

1. A method for propagating and/or differentiating mammalian mesenchymal cells from the umbilical cord or from the umbilical cord blood, the method comprising either 1) exposing mammalian mesenchymal cells from the umbilical cord or from the umbilical cord blood to periodontal ligament tissue or 2) co-culturing such mesenchymal cells with periodontal ligament tissue, to obtain cells having PDL characteristics and fulfilling at least one of the following: i) showing periodontal characteristics as evidenced with Von Kossa method in which calcium phosphate deposits are stained brown to black, ii) showing increased osteopontin and osteocalcin and at the same time decreased bone sialoprotein (bone sialoprotein II or BSP), iii) being capable of being implanted to repair and/or regenerate periodontal tissue, iv) being capable of repairing disorders such as paradentitis also called paradentosis, or periodontitis by healing of the gum line towards the teeth, and v) being accepted by the host without significant immune reaction or cell rejection.

2. A method according to claim 1, wherein the mammalian cells are propagated in a growth medium, then induced to differentiation by shifting the growth medium to a differentiation medium such as an osteoinductive medium, whereupon the cells with PDL characteristics obtained are harvested.

3. A method according to claim 1 or 2, wherein the mammalian cells are allogenic mammalian mesenchymal cells or autologous mammalian mesenchymal cells, wherein these cells by exposure to periodontal ligament protein will differentiate into PDL cells.

4. A method according to claim 3, wherein the mammalian mesenchylmal cells that are propagated are autologous mesenchymal cells and the cells obtained are capable of transdifferentiate in situ to PDL cells or to cells showing PDL characteristics.

5. A method according to any of the preceding claims, wherein the cells obtained with PDL characteristics are HLA-DR positive (HLA-DR+), Lin positive (Lin +), Thy-1 positive (Thy-1 +), Stro-1 positive (Stro-1 +), CD-13 positive, CD-44 positive, CD-90 positive, CD-73 positive and, possibly CD146/MUC 18 positive, but negative for CD31.

6. A method according to any of the preceding claims, wherein the cells obtained with PDL characteristics are CD-34 negative.

7. The method according to any one of claims 1-6, further comprising the step of manufacturing a medicament comprising the cells which have been propagated and/or differentiated, where said medicament is intended for use in a method of treating dental disorders by injecting the cells directly down to periodontal ligaments resulting in adherence of the cells to the surrounding root canal.

8. The method according to claim 7, wherein implantation of cells employs a combination of cells and scaffold(s).

## Patentansprüche

1. Verfahren zur Vermehrung und/oder Differenzierung mesenchymaler Säugerzellen aus der Nabelschnur oder aus dem Nabelschnurblut, wobei das Verfahren entweder 1) das in Kontakt bringen von mesenchymalen Säugerzellen aus der Nabelschnur oder aus dem Nabelschnurblut mit periodontalem Ligamentgewebe oder 2) Ko-Kultivierung solcher mesenchymaler Zellen mit periodontalem Ligamentgewebe umfasst, um Zellen zu erhalten, welche PDL-Merkmale aufweisen und mindestens eines der folgenden Merkmale verwirklichen: i) Aufweisen von periodontalen Merkmalen, die mit dem von Kossa Verfahren belegt werden können, bei dem Kalziumphosphatablagerungen braun bis schwarz gefärbt werden, ii) Aufweisen von erhöhtem Osteopontin und Osteocalcin und gleichzeitig vermindertem Bone Sialoprotein (Bone Sialoprotein II oder BSP), iii) implantierbar um periodontales Gewebe zu reparieren und/oder zu regenerieren, iv) Krankheiten, wie zum Beispiel Parodentitis, auch Parodontose genannt, oder Periodontitis durch Heilung der Zahnfleischlinie in Richtung der Zähne beheben zu können, und v) vom Empfänger ohne signifikante Immunreaktion oder Zellabstoßung akzeptiert werden.

2. Verfahren nach Anspruch 1, wobei die Säugerzellen in einem Wachstumsmedium vermehrt werden, danach zur Differenzierung durch Wechsel des Wachstumsmediums zu einem Differenzierungsmediums, zum Beispiel ein osteoinduktives Medium, induziert werden, woraufhin die erhaltenen Zellen mit PDL-Merkmalen gewonnen werden.

3. Verfahren nach Anspruch 1 oder 2, wobei die Säugerzellen allogene mesenchymale Säugerzellen oder autologe mesenchymale Säugerzellen sind, wobei sich diese Zellen durch in Kontakt bringen mit periodontalem Ligamentprotein zu PDL-Zellen differenzieren.

4. Verfahren nach Anspruch 3, wobei die mesenchymalen Säugerzellen, welche vermehrt werden, autologe mesenchymale Zellen sind und die erhaltenen Zellen in der Lage sind, in situ zu PDL-Zellen oder zu PDL-Merkmale aufweisende Zellen zu transdifferenzieren.

5. Verfahren nach einem der vorangehenden Ansprüche, wobei die erhaltenen Zellen mit PDL-Merkmalen HLA-DR positiv (HLA-DR+), Lin positiv (Lin +), Thy-1 positiv (Thy-1 +), Stro-1 positiv (Stro-1 +), CD-13 positiv, CD-44 positiv, CD-90 positiv, CD-73 positiv und, möglicherweise CD146/MUC 18 positiv, aber negativ für CD31 sind.

6. Verfahren nach einem der vorangehenden Ansprüche, wobei die erhaltenen Zellen mit PDL-Merkmalen CD-34 negativ sind.

7. Verfahren nach irgendeinem der Ansprüche 1-6, weiter umfassend den Schritt der Herstellung eines Medikaments umfassend die Zellen, welche vermehrt und/oder differenziert worden sind, wobei das Medikament bestimmt ist zur Verwendung in einem Verfahren zur Behandlung von Zahnerkrankungen durch Injizieren der Zellen direkt unter periodontale Ligamente, was zu einer Adhärenz der Zellen an den umgebenden Wurzelkanal führt.

8. Verfahren nach Anspruch 7, wobei bei Implantation von Zellen eine Kombination von Zellen und Gerüst(en) verwendet wird.

## Revendications

1. Procédé de reproduction et/ou de différentiation de cellules mésenchymes mammifères issues du cordon ombilical ou du sang de cordon ombilical, le procédé comprenant soit 1) l'exposition de cellules mésenchymes mammifères du cordon ombilical ou du sang de cordon ombilical vers un desmodonte ou 2) la co-culture de ces cellules mésenchymes avec un desmodonte, pour obtenir des cellules ayant des caractéristiques desmodontales et remplissant au moins une des caractéristiques suivantes: i) présentant des caractéristiques parodontales comme le montre la méthode de Von Kossa dans laquelle des dépôts de phosphates de calcium sont colorés de brun à noir, ii) présentant une ostéopontine et une ostéocalcine croissantes et simultanément une sialoprotéine osseuse décroissante (sialoprotéine osseuse II ou BSP), iii) pouvant être capables d'être implantées pour restaurer et/ou régénérer le tissu parodontal, iv) étant capables de restaurer des troubles tels que la parodontite appelée aussi parodontose, ou périodontite par la guérison de la gencive vers la dent, et v) pouvant être acceptées par l'hôte sans réaction immunitaire significative ni rejet cellulaire.

2. Procédé selon la revendication 1, **caractérisé en ce que** les cellules mammifères se reproduisent dans milieu de croissance, puis sont amenées à la différentiation en transférant le milieu de croissance vers un milieu de différentiation tel qu'un milieu ostéoinducteur, après quoi sont récoltées les cellules ayant obtenues des caractéristiques desmodontales.

3. Procédé selon les revendications 1 ou 2, **caractérisé en ce que** les cellules mammifères sont des cellules mésenchymes mammifères allogéniques ou des cellules mésenchymes mammifères autologues, **en ce que** ces cellules vont se différentier en cellules desmodontales par exposition à une protéine desmodontale.

4. Procédé selon la revendication 3, **caractérisé en ce que** les cellules mésenchymes mammifères qui sont reproduites sont des cellules mésenchymes mammifères autologues et les cellules obtenues sont capables de se transdifférentier in situ en cellules desmodontales ou en cellules présentant des carcatéristiques desmodontales.

5. Procédé selon l'une quelconque des revendications précédentes, **caractérisé en ce que** les cellules obtenues avec des caractéristiques desmodontales sont HLA-DR positive (HLA-DR+), Lin positive (Lin +), Thy-1 positive (Thy-1 +), Stro-1 positive (Stro-1 +), CD-13 positive, CD-44 positive, CD-90 positive, CD-73 positive et voire CD146/MUC 18 positive, mais négative pour CD31.

6. Procédé selon l'une quelconque des revendications précédentes, **caractérisé en ce que** les cellules obtenues avec des caractéristiques desmodontales sont CD-34 négatives.

7. Procédé selon l'une quelconque des revendications 1 à 6, comprenant en outre l'étape de fabrication d'un médicament comprenant les cellules qui ont été reproduites et/ou différentiées, où ledit médicament est destiné à être utilisé pour un procédé de traitement de troubles dentaires en injectant les cellules directement vers les desmodontes résultant de l'adhérence des cellules au canal radiculaire environnant.

8. Procédé selon la revendication 7, **caractérisé en ce que** l'implantation de cellules emploie une combinaison de cellules et de support(s).
